Europäisches Patentamt

European Patent Office

Office européen des brevets

(11) Veröffentlichungsnummer: **0 103 070**
**A2**

(12) **EUROPÄISCHE PATENTANMELDUNG**

(21) Anmeldenummer: 83105304.6

(22) Anmeldetag: 28.05.83

(51) Int. Cl.³: **B 01 D 53/04**

(30) Priorität: 16.06.82 DE 3222560

(43) Veröffentlichungstag der Anmeldung: 21.03.84
Patentblatt 84/12

(84) Benannte Vertragsstaaten: **BE DE FR GB IT NL**

(71) Anmelder: **Bergwerksverband GmbH,
Franz-Fischer-Weg 61, D-4300 Essen 13 (DE)**

(72) Erfinder: **Richter, Ekkehard, Dr.,
Schmachtenbergstrasse 89, D-4300 Essen 18 (DE)**
Erfinder: **Körbächer, Werner, Striepensweg 4,
D-4330 Mülheim/Ruhr (DE)**
Erfinder: **Knoblauch, Karl, Dr., Semperstrasse 55,
D-4300 Essen 1 (DE)**
Erfinder: **Harder, Burkhard, Dr., Am Barchenbach 12,
D-4200 Oberhausen (DE)**
Erfinder: **Giessler, Klaus, Weindorfstrasse 21,
D-4650 Gelsenkirchen (DE)**
Erfinder: **Kronauer, Peter, Adelgeundenweg 65,
D-4300 Essen (DE)**
Erfinder: **Tarnow, Ferdinand, Im Ährenfeld 41,
D-4100 Duisburg 25 (DE)**

(54) Verfahren und Vorrichtung zur Trennung von Gasen mit Adsorbentien.

(57) Bei einem Verfahren zur Abtrennung und Gewinnung von relativ stark an Adsorptionsmitteln adsorbierbaren Gasen (Produktgaskomponenten), insbesondere Methan oder Kohlendioxid, aus ansonsten im wesentlichen nur leichter adsorbierbare Gase (Abgaskomponenten) enthaltenden Gasgemischen an, insbesondere kohlenstoffhaltigen, Adsorptionsmitteln mittels Druckwechseltechnik in Adsorbern, bei dem in der Adsorptionsphase das zu trennende Ausgangsgasgemisch unter Adsorption der Produktgaskomponente(n) und Abströmen der Abgaskomponente(n) durch eine Adsorptionsmittelschicht geleitet wird, bis der Durchbruch der Produktgaskomponente(n) bevorsteht, dann die Produktgaskomponente(n) durch Druckerniedrigung und Spülen desorbiert wird (werden) und nach abgeschlossener Desorption der Gasdruck im Adsorber mit einem Gasge- misch aus dem Druckwechselprozeß für eine erneute Adsorptionsphase wieder aufgebaut wird, wird ein Produktgas hoher Reinheit und Ausbeute dadurch erhalten, daß auf Adsorptionsdruck mit zumindest zum Ende der Spülung etwa Produktgasqualität aufweisenden Produktgaskomponenten gespült wird, wobei das zunächst abströmende Gas ins Abgas und später abströmendes, an der (den) Produktgaskomponente(n) angereichertes Gas gegebenenfalls erneut dem Druckwechselprozeß wieder zugeführt wird, worauf sich die Desorptionsphase anschließt, in der unter Entspannen, insbesondere Evakuieren, des Adsorbers auf einen niedrigeren Druck ein hoch angereichertes Produktgas erhalten wird, von dem ein Teil zum Spülen dem Druckwechselprozeß wieder zugeführt und der Rest gewonnen wird.

-1-

0103070

Die Erfindung betrifft ein Verfahren gemäß dem Oberbegriff
von Patentanspruch 1 sowie eine Anlage zum Durchführen des
Verfahrens.

Man kann Kohlendioxid, Methan und andere, relativ stark an
Adsorptionsmitteln adsorbierbaren Gase (Produktgaskomponenten) aus solche enthaltenden Gasgemischen abtrennen,
indem man die Produktgaskomponenten an, insbesondere kohlenstoffhaltigen, Adsorptionsmittel adsorbiert. Bisher gelang es jedoch nicht, diese adsorbierten Produktgaskomponenten in reiner Form, beispielsweise mit Konzentrationen
von über 99,5 Vol.-%, bei der Desorption zurückzugewinnen.
Andererseits fallen bei einigen technischen Anwendungsfällen aber solche Produktgaskomponenten in Mengenanteilen
von etwa 15 bis 50 Vol.-% an, so daß ihre Gewinnung durchaus interessant wäre, so z. B.:

- Methan aus Grubengas
- Acethylen aus einem Acethylen/Wasserstoffkreislaufgas
- Kohlenmonoxid aus einem Reformergas
- Kohlendioxid aus Konvertergas oder Rauchgas.

Die Schwierigkeit der Abtrennung von solchen Produktgaskomponenten aus Gasgemischen durch Adsorption liegt bisher
darin, daß bei der Desorption eines beladenen Adsorbers
auch die gleichzeitig adsorbierten, anderen Gaskomponenten
des Ausgangsgasgemisches freigesetzt werden. Auf diese
Weise sind in den desorbierten Gasen (Produktgas) auch die
im Ausgangsgasgemisch enthaltenen Komponenten mehr oder
weniger als Verunreinigungen enthalten.

Der Erfindung liegt deshalb die Aufgabe zugrunde, Gasgemische, welche die Produktgaskomponente(n) von unter 15
Vol.-% bis über 50 Vol.-% enthalten, selektiv an, insbesondere kohlenstoffhaltigen, Adsorptionsmitteln so zu

...

trennen, daß eine Produktgaskomponente(n) in einer möglichst hohen Reinheit von z. B. über 99,5 Vol.-% und in möglichst hoher Ausbeute erhalten wird (werden).

Diese Aufgabe wird hinsichtlich eines Verfahrens durch die kennzeichnenden Merkmale von Patentanspruch 1 gelöst.

Die Druckwechseltechnik zur Trennung und Gewinnung von Gaskomponenten an Adsorptionsmitteln in Adsorbern beinhaltet bekanntlich einen zyclischen Prozeß, bei dem innerhalb eines Zyklus, der auf Adsorptionsdruck beginnt, zunächst in einer Adsorptionsphase aus dem die Adsorptionsmittelschicht durchströmenden Ausgangsgasgemisch die stark adsorbierbaren Gase im Eingangsbereich der Adsorptionsmittelschicht adsorbiert werden. Vor Beginn der Beladung strömen die leichter oder kaum adsorbierbaren Gaskomponenten am Ende der Adsorptionsmittelschicht ab, weil diese Komponenten auf ihren ursprünglichen Adsorptionsplätzen von stärker adsorbierbaren Gaskomponenten verdrängt worden sind. Hieran schließt sich ein Desorptionsschritt an, in dem durch Druck- oder Partialdruckerniedrigung im Adsorber die adsorbierten Gaskomponenten möglichst weitgehend desorbiert werden und abströmen. Abschließend erfolgt ein Druckaufbau auf Adsorptionsdruck und der Zyklus beginnt danach von neuem. All diese Phasen können mehrstufig ablaufen und insbesondere ist es bekannt, die Desorption durch Spülgasanwendung möglichst vollständig durchzuführen.

Als Produktgaskomponenten werden erfindungsgemäß diejenigen Gaskomponenten des Ausgangsgasgemisches verstanden, die relativ am stärksten und deshalb im Eingangsbereich der Adsorptionsmittelschicht adsorbieren und die leichter adsorbierbaren Gaskomponenten beim Fortschreiten der Adsorptionsfront von ihren Adsorptionsplätzen verdrängen;

...

entsprechend umgekehrtes gilt für die leichter adsorbierbaren Gaskomponenten, die der Einfachheit halber als Abgaskomponenten bezeichnet werden. Dementsprechend beinhaltet das Produktgas einen höheren Volumenanteil an Produktgaskomponenten als das Ausgangsgasgemisch, während das Abgas entsprechend stark an Produktgaskomponenten abgereichert ist.

Der erfindungsgemäße Spülschritt (Ziff. b) wird nun mit
einem Gasgemisch aus dem Druckwechselprozeß durchgeführt,
welches an Produktgaskomponenten angereichert ist; hierbei
kann das Spülgas im Laufe der Spülphase konzentrationsmäßig an der Produktgaskomponente allmählich oder stufenweise zunehmen, in jedem Fall ist aber zum Ende der Spülphase
mit einem Gas von etwa Produktgasqualität zu spülen. Das
beste Ergebnis wird erzielt, wenn die gesamte Spülung mit
einem Gasgemisch von Produktgasqualität erfolgt. Dieser
Spülschritt, der eigentlich mehr ein zweiter Adsorptionsschritt ist, da er auf Adsorptionsdruck stattfindet, führt
also dazu, daß die noch nicht von den Produktgaskomponenten besetzten Adsorptionsplätze unter Verdrängung der dort
adsorbierten Abgaskomponenten mit Produktgaskomponenten
aufgefüllt werden. Auf diese Weise ist am Ende der Spülphase das gesamte Adsorptionsmittel mit Produktgaskomponenten belegt, so daß bei der darauffolgenden Druckerniedrigung in der Desorptionsphase nur noch unwesentlich verunreinigtes Produktgas abströmt. Für eine hohe Ausbeute an
der Produktgaskomponente versteht es sich, daß die eigentliche Adsorption dann beendet wird, wenn die Produktgaskomponente kurz vor dem Durchbruch steht oder gerade
durchgebrochen ist, und es versteht sich weiterhin, daß
die "Spülung" höchstens so lange betrieben wird, bis der
Durchbruch von zunächst nur einigen Prozent an Produktgaskomponente annähernd vervollständigt ist, d. h. annähernd
100 % Produktgas aus der Adsorptionsmittelschicht abströ-

...

men. Das bei der "Spülung" abströmende Gasgemisch kann
nach einer längeren Spüldauer eine höhere Produktgaskonzentration als das Ausgangsgasgemisch aufweisen und wird
in diesem Falle bevorzugt dem Druckwechselprozeß wieder
zugeführt, und zwar für die Adsorptionsphase oder die
Spülphase; zumindest das gegen Ende der Spülphase abströmende Gasgemisch eignet sich jedoch am besten zum Einsatz
für die Adsorptions- bzw. Spülphase in einem parallelen
Adsorber, weil dann die Produktgaskonzentration schnell
bis auf Produktgasqualität steigt und so den Anforderungen
des "Spülschrittes" in besonderem Maße genügt.

Es versteht sich, daß beim Anfahren einer Anlage nach der
Erfindung eine gewisse Einlaufzeit erforderlich ist, in
der sich die einzelnen Mengenströme und Gaskonzentrationen
noch verschieben; entscheidend für die Erfindung ist der
nach einer gewissen Zahl von Zyklen erreichte Gleichgewichtszustand.

Besonders vorteilhaft für eine schnelle und hohe Aufnahmefähigkeit des Adsorptionsmittels für die Produktgaskomponente(n) bei der Adsorptionsphase sowie eine schnelle und
möglichst vollständige Desorption der Produktgaskomponente(n) bei der Desorption. Dies wird erfindungsgemäß dadurch erreicht, daß der Druckwechselprozeß im wesentlichen
adiabatisch geführt wird.

Mit dem Verfahren der Erfindung lassen sich alle relativ
stark adsorbierbaren Gase, insbesondere Methan und Kohlendioxid, enthaltende Gasgemische, beispielsweise solche mit
Sauerstoff, Stickstoff, Kohlenmonoxid, Wasserstoff, Helium, Argon oder anderen, an dem, vorzugsweise kohlenstoffhaltigen, Adsorptionsmittel schwach adsorbierbaren Gas, zu
einem Produktgas hoher Reinheit und Ausbeute aufarbeiten.
Überraschenderweise ist es auch unschädlich, wenn im Aus-

...

Ausgangsgasgemisch auch Spuren, z. B. unter 1 Vol.-%, von noch stärker adsorbierbaren Gaskomponenten als der (den) Produktgaskomponente(n) - das Produktgas kann auch ein besonders interessierendes Gasgemisch aus mehreren etwa gleich stark adsorbierbaren Gaskomponenten sein - vorhanden sind, weil diese in jedem Falle adsorbiert, meistens aber durch Druckerniedrigung allein nicht desorbiert werden, so daß sie das Desorptionsgas (Produktgas) nicht verunreinigen, sondern z. B. in einem sich an die Desorptionsphase anschließenden Spülschritt entfernt werden können; man kann auf diesen Spülschritt aber verzichten, wenn die Spurenverunreinigungen nur sehr gering sind und die dadurch hervorgerufene Kontamination des Adsorptionsmittels in vertretbaren Grenzen bleibt, oder man verwendet einen speziellen Vorfilter für die Spurenverunreinigungen. In jedem Fall muß das Adsorptionsmittel - in an sich bekannter Weise - hinsichtlich seiner Adsorptionseigenschaften auf die Produktgaskomponente(n) abgestimmt sein.

Das erfindungsgemäße Verfahren kann auch z. B. bei der Stickstoffgewinnung aus Luft an Zeolithen mit Druckwechseltechnik durchgeführt werden, da hierbei der Stickstoff stärker als der Sauerstoff adsorbiert, und er im Desorptionsschritt gewonnen wird.

Es ist allgemein schon vor der Erfindung bekannt gewesen, Gasgemische unter Ausnutzung der unterschiedlichen Adsorptions- und Desorptions-Eigenschaften an Adsorptionsmitteln durch Druckwechsel oder Temperaturwechsel zu trennen. Der Erfindung liegt demgegenüber eine Arbeitsweise zugrunde, mit der es gelingt, daß vor Beginn der Desorptionsphase praktisch keine anderen Gasbestandteile außer der (dem) Produktgaskomponente(n) im Adsorber vorhanden sind.

...

Diese vollständige Beladung der Produktgaskomponente wird bei der Erfindung also dadurch erreicht, daß nach der Adsorptionsphase der Adsorber mit einem Teil des Produktgases, das bevorzugt über 99,5 Vol.-% Produktgaskomponente enthält, gespült wird und das dabei den Adsorber verlassende, an Produktgaskomponente angereicherte Gas (Rücklaufgas), dessen Produktgaskomponente-Konzentration zwischen dem Ausgangsgas und dem Produktgas liegt, erneut dem Druckwechselprozeß zugeführt wird. Für zumindest das Ende der Spülphase wird gemäß der Erfindung ein Anteil des Produktgases verwendet. Dieser sollte über etwa 10 Vol.-% sein und etwa 90 Vol.-% des Produktgases nicht überschreiten. Die Höhe des Anteiles hängt von der Konzentration der Produktgaskomponente(n) im Ausgangsgas ab; er ist desto höher, je niedriger die Produktgaskomponenten-Konzentration hier ist. Ein vorteilhaftes, weiteres Merkmal der Erfindung besteht darin, daß während der Spülung des Adsorbers mit Produktgas in den ersten zwei Dritteln der Spülphase das den Adsorber verlassende Gas in das Abgas gegeben wird und nur das im letzten Drittel anfallende, an Produktgaskomponenten angereicherte Gas (Rücklaufgas) zur erneuten Adsorption oder Spülung in einem nachfolgenden Zyklus eingesetzt wird (Patentanspruch 3); hierdurch wird bei hoher Ausbeute die Zykluszeit vermindert.

In besonderen Fällen kann es sich als ausreichend erweisen, die Spülung nicht bis zum vollständigen Durchbruch der Produktgaskomponente zu betreiben. Dann wird das bei der Desorption des Adsorbers gewonnene Gasgemisch zu Beginn der Desorptionsphase zum Spülen eines anderen Adsorbers verwendet, und erst nach einiger Zeit wird das in einer Reinheit von über 99,5 Vol.-% gewonnene Produktgas in einen Anteil zum Spülen und in einen in die Produktgasleitung gehenden Teilstrom aufgeteilt.

Außerdem ist es für die gewünschte vollständige Beladung mit der (den) Produktgaskomponente(n) wichtig, daß der Druckaufbau bei Ausgangsgasen mit z. B. weniger als etwa 15 Vol.-% Kohlendioxid oder weniger als etwa 10 Vol.-% Methan mit Ausgangsgas selbst, dagegen bei Ausgangsgasen mit höherer Konzentration der Produktgaskomponente(n) mit dem Abgas der Gasgemischtrennung durchgeführt wird. Außerdem wird nach einem weiteren, bevorzugten Merkmal der Erfindung in der Adsorptionsphase nach dem Druckaufbau nur für eine begrenzte Zeitspanne das zu trennende Ausgangsgas, später dagegen ein an der Produktgaskomponente angereichertes Gas, aus der Spülphase eines anderen Zyklus oder eine Mischung aus beiden eingeleitet. Ziel aller vorgenannten Maßnahmen ist es, daß bei der Druckentspannung oder Evakuierung - die Adsorption wird vorteilhaft auf dem natürlich vorhandenen Druckniveau des Ausgangsgases vorgenommen - in der Desorptionsphase ein Produktgas mit vorzugsweise mindestens 99,5 Vol.-% Produktgaskomponente aus dem Adsorber austritt.

Die Spülung des Adsorbers kann bei der beschriebenen Arbeitsweise im Gleichstrom zur Adsorption erfolgen, dagegen kann die Desorption des Adsorbers im Gleich- oder Gegenstrom zur Adsorption oder auch, besonders schnell, von beiden Enden des Adsorbers gleichzeitig erfolgen.

Die Zeitdauer für einen vollständigen Adsorptions- und Desorptionsschritt sollte nach der Erfindung etwa so aufgeteilt sein, daß der Druckaufbau und die Beladung des Adsorptionsmittels mit Produktgaskomponente in der Adsorptionsphase etwa zwischen halb bis doppelt so lang wie die Spülphase oder die Desorptionsphase dauern. Es hat sich nämlich gezeigt, daß beispielsweise in einer Vier-Adsorber-Anlage die Adsorptionsphase etwa doppelt so lang ist, wie die Spül- oder Desorptionsphase. In einer Drei-Adsor-

...

ber-Anlage genügen in der Regel zwar gleiche Zeiten für alle drei Phasen, jedoch ist der Wirkungsgrad niedriger als bei der Vier-Adsorber-Anlage.

Weitere Einzelheiten des erfindungsgemäßen Verfahrens und der hierfür erforderlichen Anlage zur Durchführung des Verfahrens werden anhand der Zeichnung näher erläutert. Es zeigen:

Fig. 1          eine Anlage zur Durchführung des Verfahrens mit vier Adsorbern

Fig. 2          eine graphische Darstellung des zeitlichen Ablaufes der Phasen in den vier Adsorbern der Fig. 1

Fig. 3          eine Anlage zur Durchführung des Verfahrens mit drei Adsorbern

Fig. 4          eine graphische Darstellung des zeitlichen Ablaufes der Phasen in den drei Adsorbern der Figur 3

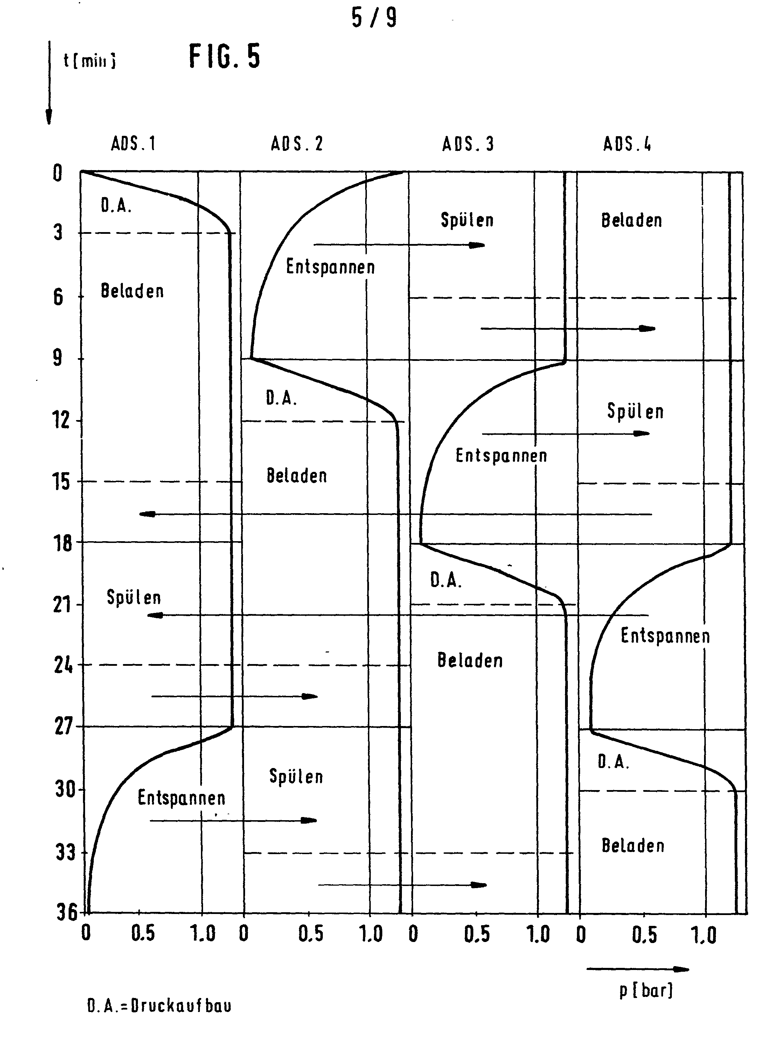

Fig. 5          ein Diagramm zum Druckverlauf in vier Adsorbern

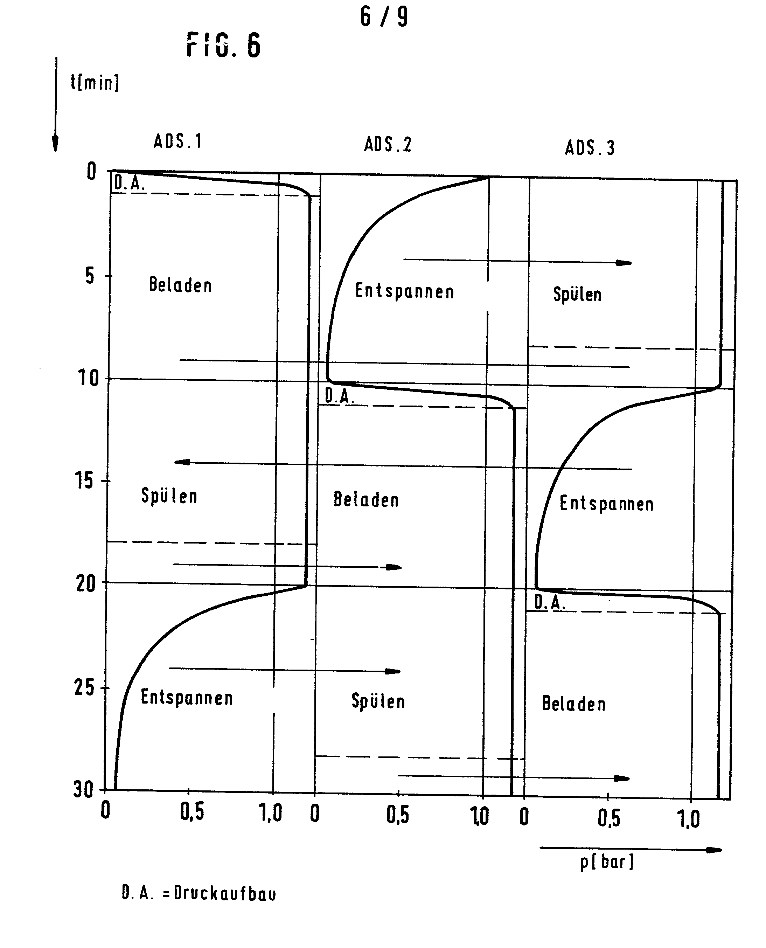

Fig. 6          ein Diagramm zum Druckverlauf in drei Adsorbern

Fig. 7          eine weitere Anlage zur Durchführung des Verfahrens mit vier Adsorbern

Fig. 8          eine graphische Darstellung des zeitlichen Ablaufes in den vier Adsorbern der Fig. 7

Fig. 9          einen Schaltplan für die Steuerung der Ventile der vier Adsorber der Fig. 7 in einem Zyklus

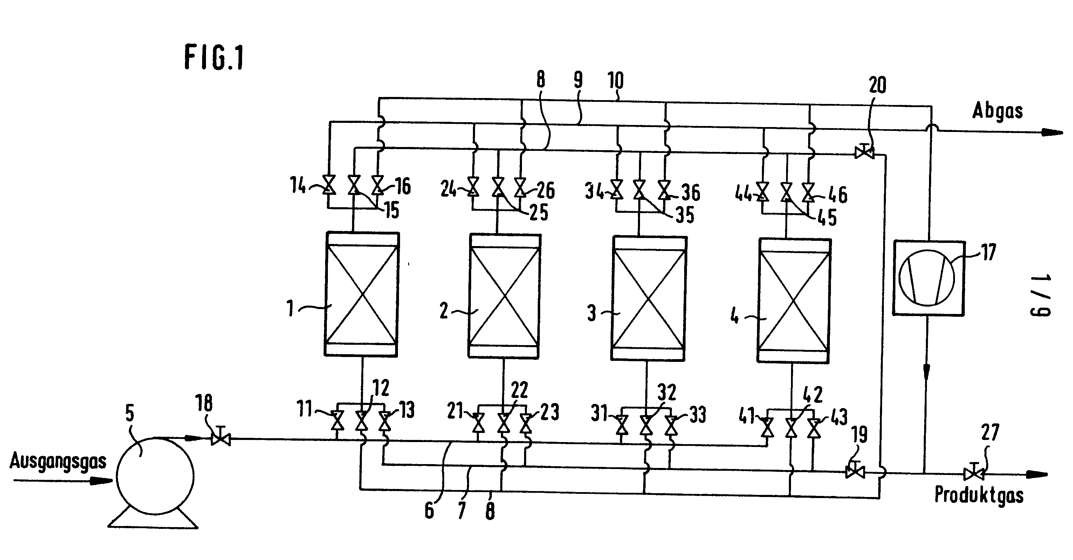

Gemäß Fig. 1 besteht die Anlage aus vier parallel geschalteten Adsorbern 1, 2, 3 und 4, die über Rohrleitung 6 sowie die Ventile 18, 11, 21, 31 und 41 mit einem Gebläse 5

· · ·

für die Förderung des Augsgangsgases sowie über die Ventile 14, 24, 34, und 44 mit einer Rohrleitung 9 für das Abgas verbunden sind. Ferner sind die Adsorber über die Ventile 16, 26, 36, und 46 über die Rohrleitung 10 mit der Vakuum-Pumpe 17 verbunden, durch die das Produktgas über das Ventil 27 abgeführt wird. Die Vakuum-Pumpe 17 ist weiterhin über die Rohrleitung 7 und die Ventile 19, 13, 23, 33 und 43 zur Überführung eines Teiles des Produktgases als Spülgas in die Adsorber verbunden, während an Kohlendioxid angereichertes Gas (Rücklaufgas) über die Rohrleitung 8 und die Ventile 15, 25, 35 45 und 20 sowie 12, 22, 32 und 42 in den jeweils nachgeschalteten Adsorber zur erneuten Adsorption zurückgeführt wird. Die Ventile 18, 19, 20 und 27 sind Drosselventile, die während des Betriebes (Ausnahme Anfahrbetrieb) in unveränderter Stellung geöffnet sind. Bei der Beschreibung der Betriebsweise werden diese Ventile nicht gesondert erwähnt.

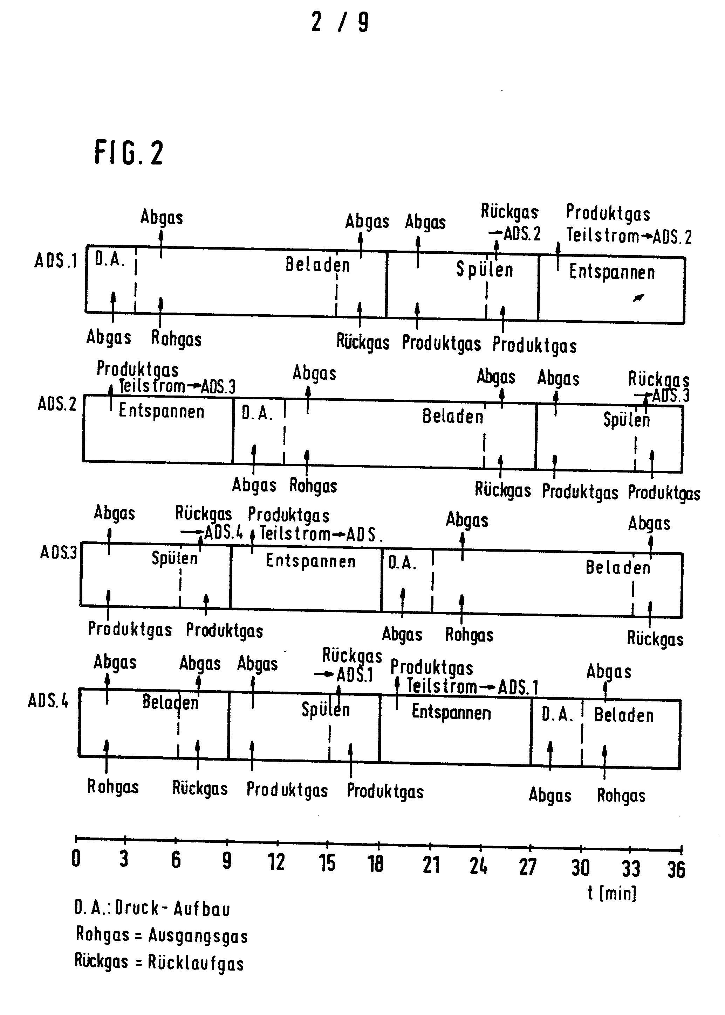

Die Inbetriebnahme einer Anlage zur Gewinnung von über 99,5 Vol.-%igem Kohlendioxid kann in der Weise erfolgen, daß zunächst durch die Ventile 11, 21, 31 und 41 sowie 14, 24, 34 und 44 alle Adsorber gleichzeitig oder nacheinander mit Ausgangsgas bis über den Durchbruch von Kohlendioxid hinaus beladen werden. Damit wird für Adsorber 1, der nachfolgend betrachtet werden soll, eine Beladung erreicht, die der nach ca. 18 min im zyklischen Betrieb (siehe Fig. 2) entspricht. Bei geschlossenem Ventil 27 und geöffnetem Ventil 36 wird Adsorber 3 evakuiert und das Produktgas, das sich während der Druckabsenkung an Kohlendioxid anreichert, wird über die Vakuumpumpe 17 und Ventile 19 und 13 durch Adsorber 1 gefördert. Dabei ist Ventil 14 zur Abgasleitung 9 geöffnet. Nach 6 min wird Ventil 14 geschlossen und das den Adsorber 1 verlassende Gasgemisch geht über die Ventile 15, 20 und 22 durch Adsorber 2 sowie Ventil 24 in die Abgasleitung 9. Nach 3 min wird Adsorber 1 in der-

...

selben Weise wie im stationären Betriebszustand evakuiert. Dieser Einlaufprozeß wird in derselben Weise, wie in Fig. 2 skizziert, durchgeführt, bis im Abgas eine mittlere Kohlendioxid-Konzentration erhalten wird, die nur unwesentlich unter derjenigen im Ausgangsgas liegt. Dann wird Ventil 27 so weit geöffnet, wie im stationären Betriebszustand erforderlich. Fig. 2 beschreibt die Betriebsweise einer Anlage mit vier Adsorbern, wobei jeder Adsorber drei Phasen durchläuft, die zum Teil noch unterteilt sein können. Nachfolgend wird nur der Zustand des ersten Adsorbers nach einem Druckaufbau mit Ausgangsgas für alle drei Phasen beschrieben. Entsprechendes gilt für alle drei anderen Adsorber.

In der ersten Phase erfolgt zuerst über 3 min (die Zahlenwerte der Zeitangaben sind beispielhaft) der Druckaufbau mit dem Ausgangsgas, wozu Ventil 11 geöffnet ist. Anschließend wird der Adsorber 1 12 min beladen. Dabei strömt Ausgangsgas bei geöffneten Ventilen 11 und 14 durch den Adsorber 1. Im wesentlichen verläßt kohlendioxidarmes Gas den Adsorber 1 und strömt in die Abgasleitung 9. Anschließend durchströmt mit Kohlendioxid angereichertes Gas (Rücklaufgas), das in der zweiten Phase des vierten Adsorbers diesen verläßt, in den ersten Adsorber bei geöffneten Ventilen 12 und 14 während 3 min. Dadurch erhöht sich die Kohlendioxid-Konzentration innerhalb des Adsorbers 1 geringfügig. Auch zu diesem Zeitpunkt ist noch kein Druchbruch von Kohlendioxid beim Adsorber 1 zu beobachten.

In der zweiten Phase wird mit der Kohlendioxid-Spülung begonnen, die mit produziertem Kohlendioxid (Produktgas) erfolgt, das bei der Desorption in Adsorber 4 anfällt. Die Spülung ist in zwei Abschnitte unterteilt. Im ersten Abschnitt verläßt bei geöffneten Ventilen 13 und 14 während 6 min ein Gasgemisch den Adsorber, dessen Kohlendioxid-Konzentration unter der des Ausgangsgases liegt. Hat die Massenübergangszone der Kohlendioxid-Spülung die Massenübergangzone der vorhergehenden Ausgangsgasbeladung erreicht, erfolgt der gemeinsame Durchbruch beider Massenübergangszonen am Adsorberausgang. Dann verläßt während 3 min bei geöffneten Ventilen 13 und 15 ein Gasgemisch den Adsorber, dessen Kohlendioxid-Konzentration zwischen der des Ausgangsgases und der des Produktgases liegt. Dieses Gasgemisch wird über das geöffnete Ventil 22 in den Adsorber 2 geleitet.

Ist der Durchbruch des Kohlendioxid durch den Adsorber 1 vollständig erfolgt, wird in der dritten Phase durch Evakuieren des Adsorbers 1 über das geöffnete Ventil 16 mit Hilfe der Vakuumpumpe 17 mit der Desorption begonnen, die etwa 9 min dauert. Ein Teil des anfallenden Produktgases mit einer Reinheit von über 99,5 Vol.-% wird zur Spülung im Adsorber 2 bei geöffneten Ventilen 19, 23 und 25 verwendet. Der andere Teil des Produktgases wird über das Ventil 27 gewonnen. Nach Beendigung der dritten Phase beginnt der beschriebene Zyklus wieder von vorne mit dem Druckaufbau.

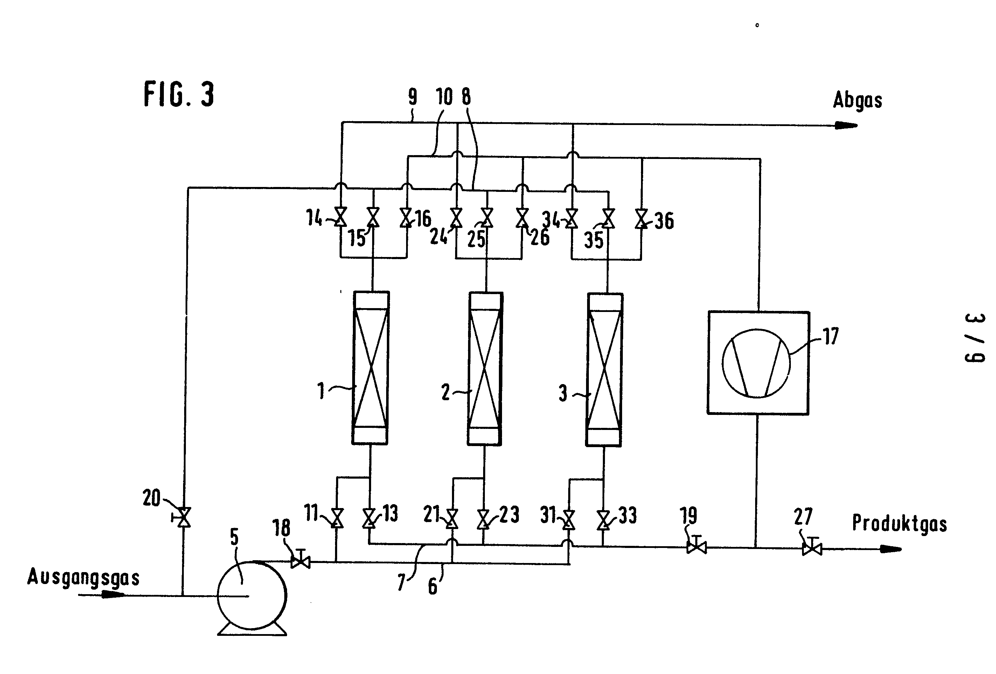

Gemäß Fig. 3 besteht die Anlage aus drei parallel geschalteten Adsorbern 1, 2 und 3, die über Rohrleitung 6 sowie die Ventile 18, 11, 21 und 31 mit einem Gebläse 5 für die Förderung des Ausgangsgases sowie über die Ventile 14, 24 und 34 mit einer Rohrleitung 9 für das Abgas verbunden sind. Ferner sind die Adsorber über die Ventile 16, 26 und 36 über die Rohrleitung 10 mit der Vakuum-Pumpe 17 verbunden, durch die das Produktgas über das Ventil 27 abgeführt wird. Die Vakuum-Pumpe 17 ist weiterhin über die Rohrleitung 7 und die Ventile 19,13, 23 und 33 zur Überführung eines Teiles des Produktgases als Spülgas in die Adsorber

...

verbunden, während an Kohlendioxid angereichertes Gas (Rücklaufgas) über die Rohrleitung 8 und die Ventile 15, 25, 35, und 20 vor dem Gebläse 5 in das Ausgangsgas zurückgeführt wird. Die Ventile 18, 19, 20 und 27 sind Drosselventile, die während des Betriebes in unveränderter Stellung geöffnet sind.

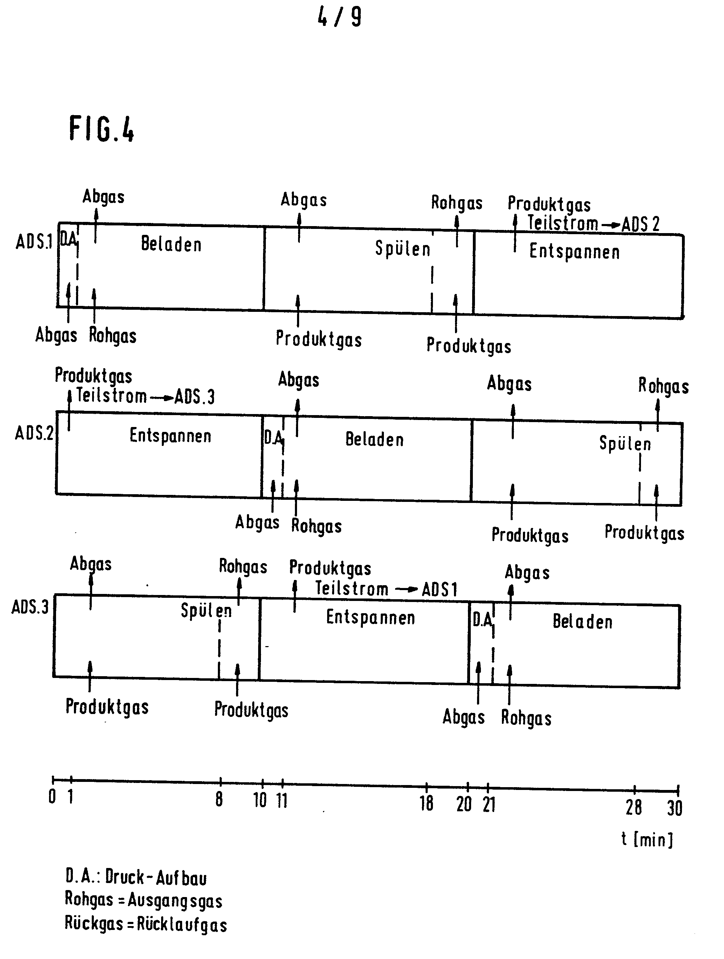

Fig. 4 beschreibt die Betriebsweise einer Anlage mit drei Adsorbern, wobei jeder Adsorber drei Phasen durchläuft, die zum Teil noch unterteilt sein können. Nachfolgend wird der Zustand des ersten Adsorbers für alle drei Phasen beschrieben. Entsprechendes gilt für die beiden anderen Adsorber.

In der ersten Phase erfolgt zuerst über 1 min (die Zahlenwerte der Zeitangaben sind beispielhaft) der Druckaufbau mit dem Ausgangsgas, wozu Ventil 11 geöffnet ist. Anschließend wird der Adsorber 1 9 min bei geöffneten Ventilen 11 und 14 beladen.

In der zweiten Phase wird mit Kohlendioxid-Produktgas gespült. Dazu sind während der ersten 8 min die Ventile 13 und 14 geöffnet. Während dieser Zeit verläßt den Adsorber ein Gasgemisch mit Kohlendioxid-Konzentrationen, die unter der des Ausgangsgases liegen. Während weiterer 2 min der Spülphase erfolgt bei geöffneten Ventilen 13 und 15 der Kohlendioxid-Durchbruch, weshalb das austretende Gasgemisch über Ventil 20 in das Ausgangsgas zurückgeführt wird.

In der dritten Phase erfolgt die Desorption bei geöffnetem Ventile 16 mit der Vakuum-Pumpe 17. Ein Teil des Produktgases wird bei geöffneten Ventilen 23 und 24 bzw. später 23 und 25 zur Spülung im Adsorber 2 verwendet. Der andere Teil wird über das Ventil 27 gewonnen. Danach beginnt ein neuer Zyklus wieder von vorne mit dem Druckaufbau.

...

Die folgenden Beispiele zeigen die Stoffbilanz während eines Zyklus für sechs Kohlendioxid- bzw. Methan-Anlagen, und zwar Tabelle 1 für eine Vier-Adsorber-Anlage, Tabelle 2 für eine Drei- Adsorber-Anlage, Tabellen 3 bis 6 für Vier-Adsorber-Anlagen.

Bei den nachfolgenden Beispielen wurde als Adsorptionsmittel ein sogenannter geformter Adsorptionskoks mit folgenden Eigenschaften eingesetzt:

Spezifische Oberfläche nach Brunauer, Emmet und Teller: 1.100 m²/g
Abmessungen: Durchmesser 3 mm
             Länge     4 mm
Schüttgewicht: 470 kg/m³
Scheinbare Dichte: 784 kg/m³

Alle Beispiele wurden einmal mit $CO_2$ und einmal mit $CH_4$ als Produktgaskomponente durchgeführt - die Werte und Angaben in Klammern gelten für die $CH_4$-Versuche, die ohne Klammern für die $CO_2$-Versuche bzw. für beide gleichzeitig.

Beispiel 1

Jeder Adsorber, Abmessungen: Höhe 2,5 m, Durchmesser 0,71 m, der Vier-Adsorber-Anlage hat ein Fassungsvermögen von 1m³, das mit Adsorptionskoks gefüllt ist. Nach einem Druckaufbau von 3 min Dauer auf 1.17 - 1.2 (1,20 - 1,25) bar folgt die Adsorption von 15 min Dauer, gefolgt von einer Spülung und Desorption bei einem Vakuum von $\leq$ 15 (60) mbar von jeweils 9 min Dauer.

...

Das Ausgangsgas hat eine Konzentration von 16,7 (37,3) Vol.-% Kohlendioxid (Methan) und 83,3 (62,7) Vol.-% Stickstoff. Hieraus ergibt sich bei einem Ausgangsgasdurchsatz von 197,27 (77,54) Nm³/h ein Netto-Produktgas-Gewinn von 27,53 (26,60) Nm³/h Kohlendioxid (Methan) mit einer Reinheit von 99,9 Vol.-%. Hieraus errechnet sich ein Wirkungsgrad der Vier-Adsorber-Anlage von 0,84 (0,92). Die Abgasmenge beträgt 169,73 (50,93) Nm³/h mit einer Kohlendioxid(Methan)-Konzentration von 3,18 (4,54) Vol.-%.

(Nm³/h = Normkubikmeter pro Stunde)

Tabelle 1

Ausgangsgas: 16,7 (37,3) Vol.-% $CO_2$ ($CH_4$), 83,3 (62,7) Vol.-% $N_2$

Adsorber: 1 m³

Adsorptionsmittel: Kohlenstoffhaltiges Adsorptionsmittel B.E.T. 1100 (m²/g)

(B. E. T. = Oberfläche nach Brunauer, Emmet und Teller)

| Phase | Ausgangsgas (Nm³) | $CO_2$ ($CH_4$) (Nm³) | $N_2$ (Nm³) |
|---|---|---|---|
| 1a. Druckaufbau (Ausgangsgas, Abgas) | 5,10 (3,29) | 0,85 (0,15) | 4,25 (3,14) |
| 1b. Adsorption | 24,49 (11,63) | 4,09 (4,34) | 20,40 (7,29) |
| Ausgangsgas (1a. + 1b.) (1b.) | 29,59 (14,92) | 4,94 (4,34) | 24,65 (10,43) |
| 2. Spülung (Produktgas) | – | 18,37 (6,39) | – |
| 3. Desorption (Produktgas Brutto) | – | 22,50 (10,38) | – |
| Netto-Produktgas (3. - 2.) | – | 4,13 (3,99) | – |
| Abgas | 25,46 (7,64) | 0,81 (0,35) | 24,65 (7,29) |

$$\text{Wirkungsgrad } \eta = \frac{\text{Netto-Produktgas (Nm}^3)}{CO_2 \text{ (Ausgangsgas) (Nm}^3)} = \frac{4,13 (3,99)}{4,94 (4,34)} = 0,84 (0,92)$$

. . .

Beispiel 2

Jeder Adsorber der Drei-Adsorber-Anlage hat ein Fassungsvermögen von 1 m³, das mit Adsorptionskoks gefüllt ist.
Nach einem Druckaufbau von 1 min Dauer auf 1.17 - 1.2
(1,20 - 1,25) bar folgt die Adsorption von 9 min Dauer,
gefolgt von einer Spülung und Desorption bei einem Vakuum
von $\leqq$ 30 (60) mbar von jeweils 10 min Dauer.

Das Ausgangsgas hat eine Konzentration von 16,7 (37,3)
Vol.-% Kohlendioxid (Methan) und 83,3 (62,7) Vol.-% Stickstoff. Hieraus ergibt sich bei einem Ausgangsgasdurchsatz
von 229,56 (70,02) Nm³/h ein Netto-Produktgas-Gewinn von
27,84 (23,22) Nm³/h Kohlendioxid (Methan) mit einer Reinheit von 99,9 Vol.-%. Hieraus errechnet sich ein Wirkungsgrad der Drei-Adsorber-Anlage von 0,73 (0,89). Die Abgasmenge beträgt 201,72 (46,80) Nm³/h mit einer Kohlendioxid-
(Methan)-Konzentration von 5,21 (6,14) Vol.-%.

Tabelle 2

Ausgangsgas: 16,7 (37,3) Vol.-% $CO_2$ ($CO_4$), 83,3 Vol.-% $N_2$
Adsorber: 1 m³
Adsorptionsmittel: Kohlenstoffhaltiges Adsorptionsmittel
B.E.T. 1100 (m²/g)

| Phase | Ausgangsgas (Nm³) | $CO_2$ ($CH_4$) (Nm³) | $N_2$ (Nm³) |
|---|---|---|---|
| 1a. Druckaufbau (Ausgangsgas, Abgas) | (3,42) | (0,21) | (3,21) |
| 1b. Adsorption (Ausgangsgas) | 38,26 (11,67) | 6,39 (4,35) | 31,87 (7,32) |
| 2. Spülung (Produktgas) | - | 18,42 (6,55) | - |
| 3. Desorption (Produktgas Brutto) | - | 23,06 (10,42) | - |
| Netto-Produktgas (3. - 2.) | - | 4,64 (1,87) | - |
| Abgas | 33,62 (7,80) | 1,75 (0,48) | 31,87 (7,32) |

$$\text{Wirkungsgrad} = \frac{\text{Netto-Produktgas (Nm}^3)}{CO_2 \text{ (Ausgangsgas) (Nm}^3)} = \frac{4,64 (3,87)}{6,39 (4,35)} = 0,73 (0,89)$$

...

Beispiel 3

Jeder Adsorber der Vier-Adsorber-Anlage hat ein Fassungsvermögen von 1 m³, das mit Adsorptionskoks gefüllt ist. Nach einem Druckaufbau von 3 min Dauer auf 1,17 - 1,20 (1,05 - 1,10) bar folgt die Adsorption von 15 min Dauer, gefolgt von einer Spülung und Desorption bei einem Vakuum von $\leqq$ 50 (60) mbar von jeweils 9 min Dauer.

Das Ausgangsgas hat eine Konzentration von 45,7 (12,5) Vol.-% Kohlendioxid (Methan) und 54,3 (84,5) Vol.-% Stickstoff. Hieraus ergibt sich bei einem Ausgangsgasdurchsatz von 172,14 (72,67) Nm³/h ein Netto-Produktgas-Gewinn von 69,74 (8,27) Nm³/h Kohlendioxid (Methan) mit einer Reinheit von 99,9 Vol.-%. Hieraus errechnet sich ein Wirkungsgrad der Vier-Adsorber-Anlage von 0,88 (0,91). Die Abgasmenge beträgt 102,40 (62,40) Nm³/h mit einer Kohlendioxid (Methan)-Konzentration von 8,7 (1,24) Vol.-%.

Tabelle 3

Ausgangsgas: 45,7 (12,5) Vol.-% $CO_2$ ($CH_4$), 54,3 (8,75) Vol.-% $N_2$ (CO)

Adsorber: 1 m³

Adsorptionsmittel: Kohlenstoffhaltiges Adsorptionsmittel B.E.T. 1.100 (m²/g)

| Phase | Ausgangsgas (Nm³) | $CO_2$ ($CH_4$) (Nm³) | $N_2$ (CO) (Nm³) |
|---|---|---|---|
| 1a. Druckaufbau (Ausgangsgas, Abgas) | 4,40 (3,84) | 0,38 (0,48) | 4,02 (3,36) |
| 1b. Adsorptions (Ausgangsgas) | 25,82 (7,06) | 11,80 (0,88) | 14,02 (6,18) |
| 2. Spülung (Produktgas) | – | 12,35 (8,49) | – |
| 3. Desorption (Produktgas Brutto) | – | 23,81 (9,79) | – |
| Netto-Produktgas (3. – 2.) | – | 10,46 (1,24) | – |
| Abgas | 15,36 (9,66) | 1,34 (0,12) | 14,02 (9,54) |

$$\text{Wirkungsgrad } \eta = \frac{\text{Netto-Produktgas (Nm}^3)}{\text{(Ausgangsgas) (Nm}^3)} = \frac{10,46(1,24)}{11,80(1,36)} = 0,88(0,91)$$

· · ·

0103070

Beispiel 4

Jeder Adsorber der Vier-Adsorber-Anlage hat ein Fassungsvermögen von 1 m³, das mit Adsorptionskoks gefüllt ist.
Nach einem Druckaufbau von 3 min Dauer auf 1.17 - 1.2
(1,10-1,15) bar folgt die Adsorption von 15 min Dauer, gefolgt von einer Spülung und Desorption bei einem Vakuum
von $\leqq$ 40 (60) mbar von jeweils 9 min Dauer.

Das Ausgangsgas hat eine Konzentration von 33,1 (52,9)
Vol.-% Kohlendioxid (Methan) und 66,9 (47,1) Vol.-% Kohlenmonoxid (Wasserstoff). Hieraus ergibt sich bei einem
Ausgangsgasdurchsatz von 171,61 (68,20) Nm³/h ein Netto-
Produktgas-Gewinn von 47,14 (33,67) Nm³/h Kohlendioxid
(Methan) mit einer Reinheit von 99,9 Vol.-%. Hieraus errechnet sich ein Wirkungsgrad der Vier-Adsorber-Anlage von
0,83 (0,93). Die Abgasmenge beträgt 124,47 (34,53)Nm³/h
mit einer Kohlendioxid(Methan)-Konzentration von 7,8
(7,05) Vol.-%.

Tabelle 4

Ausgangsgas: 33,1 (52,9) Vol.-% $CO_2$ ($CH_4$), 66,9 (47,10) Vol.-% CO ($H_2$)

Adsorber: 1 m³

Adsorptionsmittel: Kohlenstoffhaltiges Adsorptionsmittel B.E.T. 1.100 (m²/g)

| Phase | Ausgangsgas (Nm³) | $CO_2$ ($CH_4$) (Nm³) | CO ($H_2$) (Nm³) |
|---|---|---|---|
| 1a. Druckaufbau (Abgas) | 4,60 (2,72) | 0,36 (0,19) | 4,24 (2,53) |
| 1b. Adsorption (Ausgangsgas) | 25,74 (10,23) | 8,52 (5,41) | 17,22 (4,82) |
| 2. Spülung (Produktgas) | - | 15,78 (4,08) | - |
| 3. Desorption (Produktgas Brutto) | - | 22,85 (9,13) | - |
| Netto-Produktgas (3. - 2.) | - | 7,07 (5,05) | - |
| Abgas | 18,67 (5,18) | 1,45 (0,36) | 17,22 (4,82) |

$$\text{Wirkungsgrad } \eta = \frac{\text{Netto-Produktgas (Nm}^3)}{\text{(Ausgangsgas) (Nm}^3)} = \frac{7,07 (5,05)}{8,52 (5,41)} = 0,83 (0,93)$$

. . .

Beispiel 5

Jeder Adsorber der Vier-Adsorber-Anlage hat ein Fassungsvermögen von 1 m³, das mit Adsorptionskoks gefüllt ist. Nach einem Druckaufbau von 3 min Dauer auf 1,30 - 1,35 (1,00 - 1,05) bar folgt die Adsorption von 15 min Dauer, gefolgt von einer Spülung bei 1 (1) bar und Desorption bei einem Vakuum von $\leq$ 15 (60) mbar von jeweils 9 min Dauer.

Das Ausgangsgas hat eine Konzentration von 9,8 (22,1) Vol.-% Kohlendioxid (Methan) und 90,2 (77,9) Vol.-% Helium (Wasserstoff). Hieraus ergibt sich bei einem Ausgangsgasdurchsatz von 181,60 (65,14) Nm³/h ein Netto-Produktgas-Gewinn von 15,93 (12,8) Nm³/h Kohlendioxid (Methan) mit einer Reinheit von 99,9 Vol.-%. Hieraus erreichnet sich ein Wirkungsgrad der Vier-Adsorber-Anlage von 0,90 (0,89). Die Abgasmenge beträgt 165,67 (52,33) Nm³/h mit einer Kohlendioxid (Methan)- Konzentration von 1,12 (3,06) Vol.-%.

0103070

Tabelle 5

Ausgangsgas: 9,8 (22,10) Vol.-% $CO_2$ $(CH_4)$, 90,2 (77,9)
Vol.-% He $(H_2)$

Adsorber: 1 m³

Adsorptionsmittel: Kohlenstoffhaltiges Adsorptionsmittel
B.E.T. 1.100 (m²/g)

| Phase | Ausgangsgas (Nm³) | $CO_2$ $(CH_4)$ (Nm³) | He $(H_2)$ (Nm³) |
|---|---|---|---|
| 1a. Druckaufbau (Ausgangsgas, Abgas) | 2,61(2,97) | 0,26(0,66) | 2,35(2,31) |
| 1b. Adsorption Ausgangsgas | 24,63(6,8) | 2,41(1,5) | 22,22(5,3) |
| 2. Spülung (Produktgas) | - | 21,54(6,8) | - |
| 3. Desorption (Produktgas Brutto) | - | 23,93(8,72) | - |
| Netto-Produktgas (3. - 2.) | - | 2,39(1,92) | - |
| Abgas | 24,85(7,85) | 0,28(0,24) | 24,57(7,61) |

$$\text{Wirkungsgrad}_\eta = \frac{\text{Netto-Produktgas (Nm}^3)}{\text{(Ausgangsgas) (Nm}^3)} = \frac{2,39(1,92)}{2,67(2,16)} = 0,90(0,89)$$

...

Beispiel 6

Jeder Adsorber der Vier-Adsorber-Anlage hat ein Fassungsvermögen von 1 m³, das mit Adsorptionskos gefüllt ist.
Nach einem Druckaufbau von 3 min Dauer auf 1.25 - 1.30
(1,10 - 1,15) bar folgt die Adsorption von 15 min Dauer,
gefolgt von einer Spülung und Desorption bei einem Vakuum
von $\leqq$ 60 mbar von jeweils 9 min Dauer.

Das Ausgangsgas hat eine Konzentration von 79,3 (88,3)
Vol.-% Kohlendioxid (Methan) und 20,7 (11,7) Vol.-% Wasserstoff (Argon). Hieraus ergibt sich bei einem Ausgangsgasdurchsatz von 186,61 (77,0) Nm³/h ein Netto-Produktgas-Gewinn von 134,74 (64,94) Nm³/h Kohlendioxid (Methan)
mit einer Reinheit von 99,9 Vol.-%. Hieraus errechnet sich
ein Wirkungsgrad der Vier-Adsorber-Anlage von 0,91 (0,96).
Die Abgasmenge beträgt 51,87 (12,07) Nm³/h mit einer Kohlendioxid (Methan)-Konzentration von 26,2 (25,35) Vol.-%.

Tabelle 6

Ausgangsgas: 79,3 (88,3) Vol.-% $CO_2$ ($CH_4$), 20,7 (11,7) Vol.-% $H_2$ (Ar)

Adsorber: 1 m³

Adsorptionsmittel: Kohlenstoffhaltiges Adsorptionsmittel B.E.T. 1.100 (m²/g)

| Phase | Ausgangsgas (Nm³) | $CO_2$ ($CH_4$) (Nm³) | $H_2$ (Ar) (Nm³) |
|---|---|---|---|
| 1a. Druckaufbau (Abgas) | 2,81(1,77) | 0,73(0,45) | 2,08(1,32) |
| 1b. Adsorption (Ausgangsgas) | 27,99(11,55) | 22,25(10,2) | 5,74(1,35) |
| 2. Spülung (Produktgas) | – | 3,91(0,57) | – |
| 3. Desorption (Produktgas Brutto) | – | 24,12(10,31) | – |
| Netto-Produktgas (3. – 2.) | – | 20,12(9,74) | – |
| Abgas | 7,78(1,81) | 2,04(0,46) | 5,74(1,35) |

$$\text{Wirkungsgrad}_\eta = \frac{\text{Netto-Produktgas (Nm}^3)}{\text{(Ausgangsgas) (Nm}^3)} = \frac{20,21(9,74)}{22,25(10,2)} = 0,91(0,96)$$

...

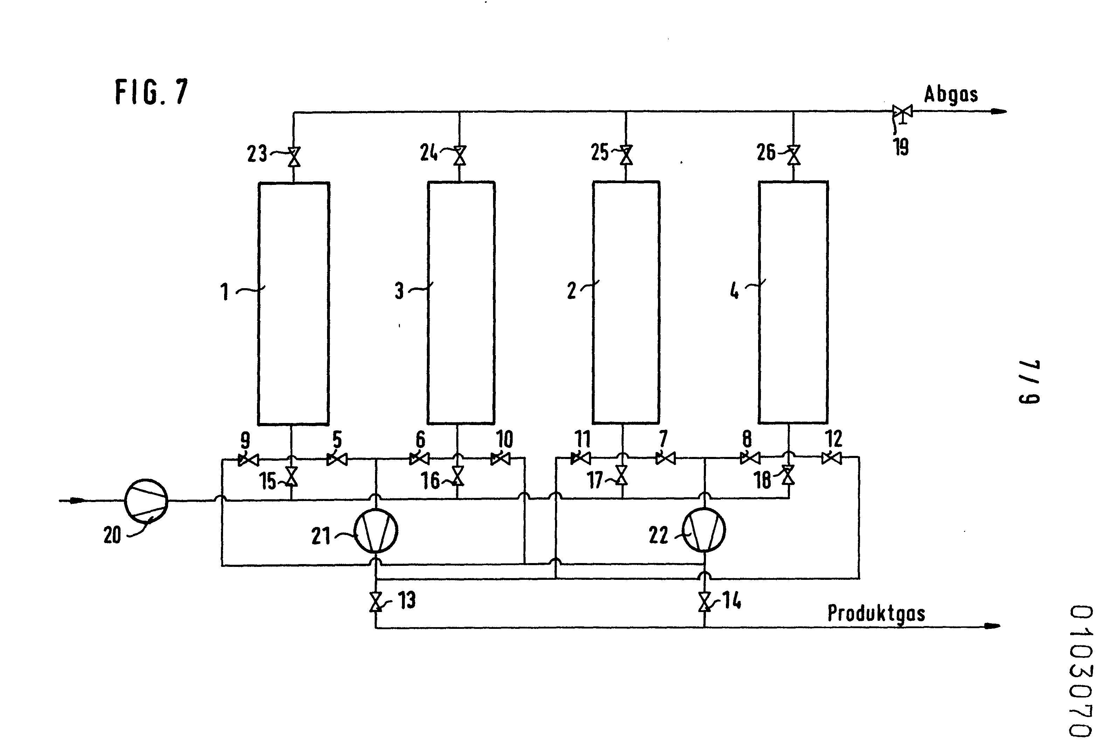

Gemäß Fig. 7 besteht eine andere Anlage aus vier in zwei Gruppen zusammengeschalteten Adsorbern, wobei Adsorber 101 mit Adsorber 102 und Adsorber 103 mit Adsorber 104 zusammenarbeitet. Die Adsorber 101, 103, 102 und 104 sind über die Ventile 115, 116, 117 und 118 mit einer Beladungspumpe 120 für die Förderung des Ausgangsgases verbunden, sowie über die Ventile 105 und 106 mit der Vakuumpumpe 121 und über die Ventile 107 und 108 mit der Vakuumpupe 122. Die Vakuumpumpe 121 ist weiterhin, zur Überführung eines Teiles des Produktgases als Spülgas (Rücklaufgas), über die Ventile 111 mit Adsorber 102 und über Ventil 112 mit Adsorber 104 verbunden, während die Vakuumpumpe 122 über Ventil 109 mit Adsorber 101 und über Ventil 110 mit Adsorber 103 verbunden ist. Über die Ventile 113 und 114 wird das Produktgas gewonnen, während über die Rückschlagventile 123, 124, 125 und 126 das Abgas die Adsorber und über Ventil 119 die Anlage verläßt.

Die Inbetriebnahme einer Anlage zur Gewinnung von über 99,5 Vol.-%igem Kohlendioxid kann in der Weise erfolgen, daß zunächst die Ventile 115, 116, 117 und 118 gleichzeitig oder nacheinander geöffnet und die Adsorber 101, 102, 103 und 104 mit Ausgangsgas beschickt werden, bis sie bis zum Gleichgewicht mit Kohlendioxid beladen sind. Danach beginnt der zyklische Betrieb der Anlage nach der in Fig. 2 und Fig. 3 dargestellten Weise.

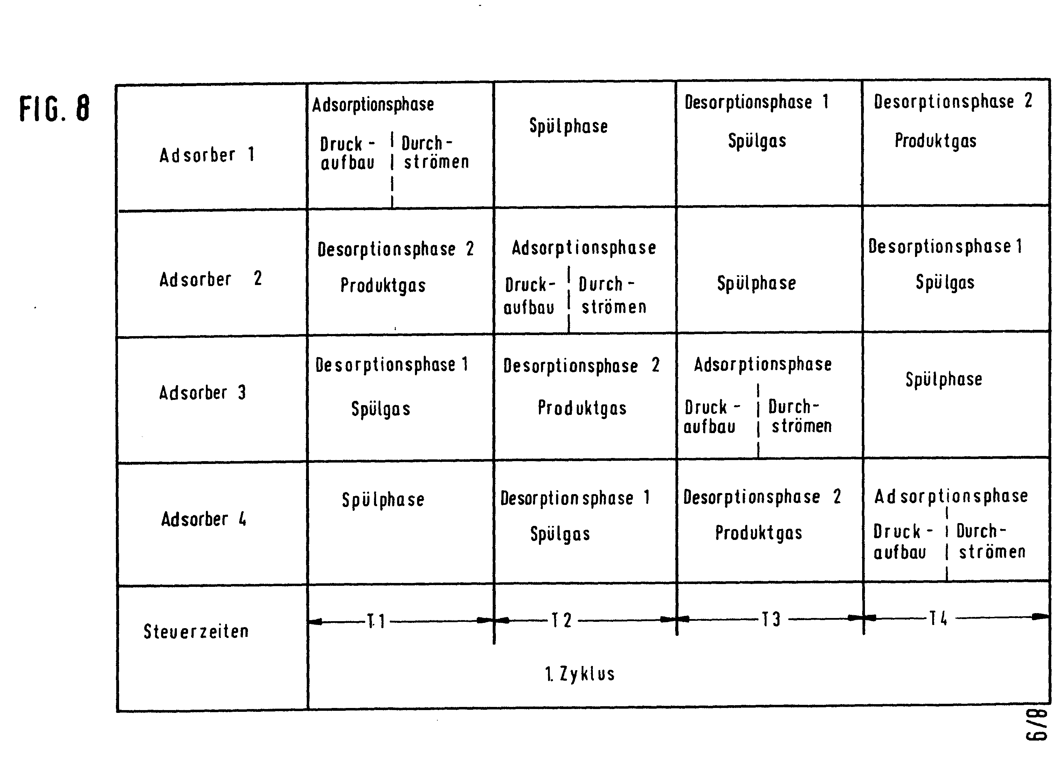

Fig. 8 beschreibt die Betriebsweise einer Anlage mit vier Adsorbern, wobei jeder Adsorber unter Berücksichtigung der beiden Desorptionsphasen zur Gewinnung von Spülgas bzw. von Produktgas vier Abschnitte durchläuft. Die Fig. 2 macht außerdem deutlich, in welchem Abschnitt sich die einzelnen Adsorber zur gleichen Zykluszeit befinden. Nachfolgend wird nur der Zustand des ersten Adsorbers, für al-

...

le vier Abschnitte beispielsweise, beschrieben. Entsprechendes gilt für alle drei anderen Adsorber.

In der ersten Phase erfolgt zuerst über 75 s (die Zahlenwerte der Zeitangaben sind beispielhaft) der Druckaufbau mit dem Ausgangsgas, wozu Ventil 15 geöffnet ist. Anschließend wird der Adsorber 101 75 s beladen. Dabei strömt Ausgangsgas durch das geöffnete Ventil 115 durch den Adsorber 101. Im wesentlichen verläßt kohlendioxidfreies Gas durch das Rückschlagventil 123 den Adsorber 101 und strömt durch das geöffnete Ventil 119 ins Abgas.

Anschließend wird in der zweiten Phase Adsorber 101 mit Desorptionsgas aus dem ersten Desorptionsphase des Adsorbers 104 mit einer mittleren Konzentration unter 99,5 Vol.-% Kohlendioxid bei geöffneten Ventilen 108 und 109 mittels der Vakuumpumpe 122 während 75 s gespült. Dadurch erhöht sich die Kohlendioxidkonzentration im Adsorber 101. Am Ende dieser Phase ist der Durchbruch von Kohlendioxid beim Adsorber 101 zu beobachten.

In der dritten Phase, der ersten Desorptionsphase, wird mit dem Evakuieren des Adsorbers 101 bei geöffneten Ventilen 105 und 111 begonnen, wobei Adsorber 102 während 75 s gespült wird. Abschließend erfolgt die Produktgasgewinnung in der zweiten Desorptionsphase mit Hilfe der Vakuumpumpe 121 bei geöffnetem Ventil 113 während weiterer 75 s. Dieses Produktgas fällt nach einer Einlaufzeit von etwa 90 min nach Inbetriebnahme der Anlage in einer Reinheit von über 99,5 Vol.-% Kohlendioxid an. Nach Beendigung der Evakuierung beginnt der beschriebene Zyklus wieder von vorne mit dem Druckaufbau.

Gemäß Fig. 3 wird gezeigt, welche Ventile während der vier Phasen in den Adsorbern 101, 102, 103 und 104 jeweils ge-

. . .

öffnet sind, um einen zyklischen Betrieb der Anlage zu erzielen.

Beide Desorptionsschritte erfolgen im Gegenstrom zur Adsorption bzw. Spülung. Hierbei werden immer von der Vakuumpumpe 21 wechselseitig Adsorber 101 und 103 evakuiert und das Desorptionsgas durch Adsorber 102 und 104 als Spülgas gedrückt, während über Ventil 113 Produktgas gewonnen wird. Desgleichen werden von der Vakuumpumpe 22 Adsorber 102 und 104 evakuiert und Adsorber 101 und 103 gespült, während das Produktgas über Ventil 114 gewonnen wird.

Die Dauer jedes Zyklus kann zwischen etwa 4 mal 50 s bis 4 mal 2.000 s betragen. Hierbei beträgt die während der ersten Desorptionsphase gewonnene Spülgasmenge für die Nachbeladung des jeweils nachgeschalteten Adsorbers das etwa 10- bis 15fache eines Adsorbervolumens.

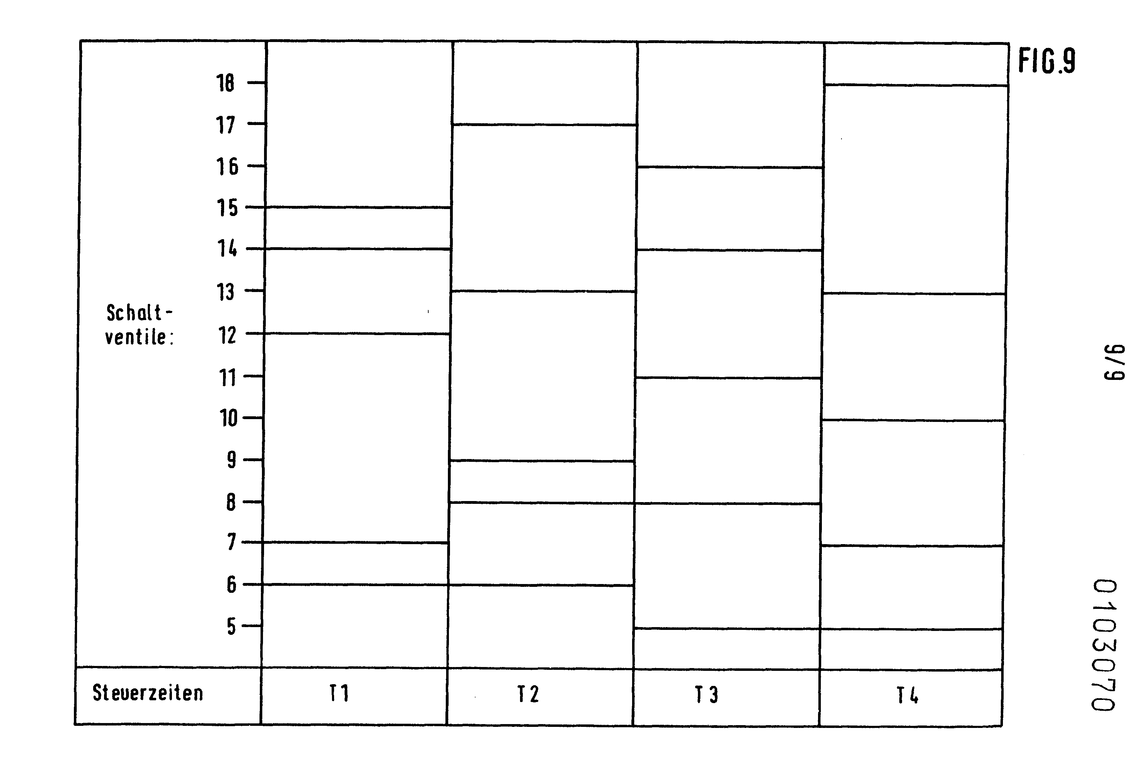

Die folgenden Beispiele zeigen die Ergebnisse einer Gastrennung bei Ausgangsgasen mit unterschiedlichen Kohlendioxidanteilen in einer Vier-Adsorber-Anlage der vorbeschriebenen Art (Fig. 7 bis 9), wobei jeder Adsorber folgende Abmessungen aufwies: Höhe 2,5 m, Durchmesser 0,71 m.

Der verwendete geformte Adsorptionskoks läßt sich folgendermaßen charakterisieren:

Spezifische Oberfläche: BET 1.100 m³/g
Abmessungen: Durchmesser 3 mm
             Länge 4 ÷ 5 mm
Schüttgewicht: 470 kg/m³
Scheinbare Dichte: 785 kg/m³

Beispiel 7

Jeder der vier Adsorber hat ein Fassungsvermögen von 1 m³, das mit Adsorptionskoks gefüllt ist. Die Adsorptionszeit beträgt 150 s. Bedingt durch die gewählte Strömungsgeschwindigkeit des Ausgangsgases entfallen hierbei 15 s auf den Druckaufbau. Es folgt die Spülung von 150 s Dauer und eine zweistufige Desorption von jeweils 150 s Dauer auf ein Vakuum von 60 mbar. Der Zyklus dauert 4 x 150 s.

Das Ausgangsgas hat eine Konzentration von 16 Vol.-% Kohlendioxid und 84 Vol.-% Stickstoff. Hieraus ergibt sich bei einem Ausgangsgasdurchsatz von 1.005 Nm³/h, ein Netto-Produktgas- Gewinn von 65 Nm³/h Kohlendioxid mit einer Reinheit von 99,5 Vol.-%.

Die Abgasmengen beträgt 940 Nm³/h mit einer Kohlendioxidkonzentration von 10,2 Vol.-%. Der Wirkungsgrad der Anlage beträgt $\eta_{(CO_2)} = 0,40$.

Beispiel 8

In der gleichen Anlage wie in Beispiel 7 wird ein Ausgangsgas mit einer Konzentration von 45 Vol.-% Kohlendioxid und 55 Vol.-% Stickstoff verarbeitet.

Die Adsorptionszeit beträgt 75 s. Bedingt durch die gewählte Strömungsgeschwindigkeit des Ausgangsgases werden hiervon 35 s für den Druckaufbau benötigt. Es folgt dann die Spülung von 75 s Dauer und eine zweistufige Desorption von jeweils 75 s Dauer auf ein Vakuum von 60 mbar. Hieraus ergibt sich bei einem Ausgangsgasdurchsatz von 537 Nm³/h

...

ein Netto-Produktgas-Gewinn von 156 Nm³/h Kohlendioxid mit
einer Reinheit von 99,8 Vol.-%.

Die Abgasmenge beträgt 381 Nm³/h mit einer Kohlendioxid-
konzentration von 22,9 Vol.-%. Der Wirkungsgrad der Anlage
beträgt $\eta$ (CO$_2$) = 0,64.

Beispiel 9

In der gleichen Anlage wie im Beispiel 1 wird ein Ausgangsgas mit einer Konzentration von 45 Vol.-% Kohlendioxid, 20 Vol.-% Wasserstoff und 35 Vol.-% Kohlenmonoxid
verarbeitet.

Die Adsorptionszeit beträgt 60 s. Hiervon entfallen 15 s
auf den Druckaufbau. Es folgt dann die Spülung von 60 s
Dauer und eine zweistufige Desorption von jeweils 60 s
Dauer auf ein Vakuum von 70 mbar. Hieraus ergibt sich bei
einem Ausgangsgasdurchsatz von 972 Nm³/h ein Netto-Produktgas-Gewinn von 209 Nm³/h Kohlendioxid mit einer Reinheit von -99,8 Vol.-%.

Die Abgasmenge beträgt 763 Nm³/h mit einer Kohlendioxid-
konzentration von 30 Vol.-%. Der Wirkungsgrad der Anlage
beträgt $\eta$ (CO$_2$) = 0,48.

Beispiel 10

In der gleichen Anlage wie im Beispiel 1 wird ein Ausgangsgas mit einer Konzentration von 66,4 Vol.-% Stickstoff, 17,6 Vol.-% Sauerstoff und 16 Vol.-% Kohlendioxid verarbeitet.

Die Adsorptionszeit beträgt 300 s. Hiervon entfallen 90 s auf den Druckaufbau. Es folgt dann die Spülung von 300 s Dauer und eine zweistufige Desorption von jeweils 300 s Dauer auf ein Vakuum von ca 50 mbar. Hieraus ergibt sich bei einem Ausgangsgasdurchsatz von 197 Nm³/h ein Netto-Produktgas-Gewinn von 24 Nm³/h Kohlendioxid mit einer Reinheit von $\geq$99,5 Vol.-%.

Die Abgasmenge beträgt 173 Nm³/h mit einer Kohlendioxidkonzentration von 5 Vol.-%. Der Wirkungsgrad der Anlage beträgt $\eta$ (CO$_2$) = 0,74.

**BERGWERKSVERBAND GMBH**

VERSUCHSBETRIEBE DER BERGBAU-FORSCHUNG GMBH

0103070

Essen, 20.05.1983

Verfahren zur Abtrennung und Gewinnung von relativ stark an Adsorptionsmitteln adsorbierbaren Gasen aus ansonsten im wesentlichen nur leichter adsorbierbare Gase enthaltenden Gasgemischen sowie Anlage zum Durchführen dieses Verfahrens

Patentansprüche

1. Verfahren zur Abtrennung und Gewinnung von relativ stark an Adsorptionsmitteln adsorbierbaren Gasen (Produktgaskomponenten), insbesondere Methan oder Kohlendioxid, aus ansonsten im wesentlichen nur leichter adsorbierbare Gase (Abgaskomponenten) enthaltenden Gasgemischen an, insbesondere kohlenstoffhaltigen, Adsorptionsmitteln mittels Druckwechseltechnik in Adsorbern, bei dem in der Adsorptionsphase das zu trennende Ausgangsgasgemisch unter Adsorption der Produktgaskomponente(n) und Abströmen der Abgaskomponente(n) durch eine Adsorptionsmittelschicht geleitet wird, bis der Durchbruch der Produktgaskomponente(n) bevorsteht, dann die Produktgaskomponente(n) durch Druckerniedrigung und Spülen desorbiert wird (werden) und nach abgeschlosse-

...

ner Desorption der Gasdruck im Adsorber mit einem Gasgemisch aus dem Druckwechselprozeß für eine erneute Adsorptionsphase wieder aufgebaut wird, dadurch gekennzeichnet, daß auf Adsorptionsdruck mit zumindest zum Ende der Spülung etwa Produktgasqualität aufweisenden Produktgaskomponenten gespült wird, wobei das zunächst abströmende Gas ins Abgas und später abströmendes, an der (den) Produktgaskomponente(n) angereichertes Gas ggf. erneut dem Druckwechselprozeß wieder zugeführt wird, worauf sich die Desorptionsphase anschließt, in der unter Entspannen, insbesondere Evakuieren, des Adsorbers auf einen niedrigeren Druck ein hoch angereichertes Produktgas erhalten wird, von dem ein Teil zum Spülen dem Druckwechselprozeß wieder zugeführt und der Rest gewonnen wird.

2. Verfahren nach Anspruch 1, dadurch gekennzeichnet, daß ein Anteil von 10 - 90 Vol.-% des Produktgases für die Spülung verwendet wird.

3. Verfahren nach Anspruch 1 oder 2, dadurch gekennzeichnet, daß das, insbesondere im letzten Drittel, der Spülphase abströmende, an der (den) Produktgaskomponente(n) angereicherte Gas am Ende der Adsorptionsphase oder am Anfang der Spülphase durch die Adsorptionsmittelschicht eines anderen, parallel geschalteten Adsorbers geleitet wird.

4. Verfahren nach einem oder mehreren der Ansprüche 1 bis 3, dadurch gekennzeichnet, daß der Druckaufbau im Adsorber bei Ausgangsgasgemischen mit 15 (10) oder weni-

...

ger Vol.-% Kohlendioxid (Methan) mit Ausgangsgasgemisch durchgeführt wird.

5. Verfahren nach einem oder mehreren der Ansprüche 1 bis 4, dadurch gekennzeichnet, daß die Adsorptionsphase vor dem Beginn des Durchbruches der Produktgaskomponente(n) durch die Adsorptionsmittelschicht beendet wird.

6. Verfahren nach einem oder mehreren der Ansprüche 1 bis 5, dadurch gekennzeichnet, daß die Spülung des Adsorbers im Gleichstrom zur Adsorption erfolgt.

7. Verfahren nach einem oder mehreren der Ansprüche 1 bis 6, dadurch gekennzeichnet, daß die Desorption des Adsorbers von beiden Enden des Adsorbers gleichzeitig erfolgt.

8. Verfahren nach einem oder mehreren der Ansprüche 1 bis 7, dadurch gekennzeichnet, daß der Druckaufbau und die Beladung des Adsorbers in der Adsorptionsphase etwa zwischen halb so lang bis doppelt so lang wie die Spülphase oder die Desorptionsphase dauern.

9. Anlage zur Durchführung des Verfahrens nach einem oder mehreren der Ansprüche 1 bis 8, dadurch gekennzeichnet, daß eine Vier-Adsorber-Anlage, bei der die einzelnen Adsorber (1 bis 4) phasenversetzt beladen und evakuiert werden am Eingang jedes Adsorbers (1 bis 4) mit jeweils drei Ventilen für Ausgangsgas (11, 21, 31, 41), Spülgas (13, 23, 33, 43) und angereichertes Gas (12, 22, 32, 42) sowie am Ausgang mit jeweils drei Ventilen für an-

...

4

0103070

gereichertes Gas (15, 25, 35, 45), Abgas (14, 24, 34, 44) und Produktgas (16, 26, 26, 46) versehen sind.

10. Anlage zur Durchführung des Verfahrens nach einem oder mehreren der Ansprüche 1 bis 8, dadurch gekennzeichnet, daß eine Drei-Adsorber-Anlage, bei der die einzelnen Adsorber (1 bis 3) phasenversetzt beladen und evakuiert werden, am Eingang jedes Adsorbers (1 bis 3) mit jeweils zwei Ventilen für Ausgangsgas (11, 21, 31) und Spülgas (13, 23, 33) sowie am Ausgang mit jeweils drei Ventilen für angereichertes Gas (15, 25, 35), Abgas (14, 24, 34) und Produktgas (16, 26, 36) versehen sind.

11. Anlage nach Anspruch 9 oder 10, dadurch gekennzeichnet, daß jede Anlage in der Ausgangsgasleitung (6) mit einem Gebläse (5) und in der Produktgasleitung (10) mit einer Vakuumpumpe (17) versehen ist.

12. Anlage nach Anspruch 1, dadurch gekennzeichnet, daß die Adsorber (101) und (103) sowie (102) und (104) in zwei Gruppen zusammengeschaltet sind, wobei die Beladungspumpe (120) über die Ventile (115), (116), (117) und (118) mit allen vier Adsorbern verbunden ist, während einerseits die Adsorber (101) und (103) über die Ventile (105) und (106) mit der Vakuumpumpe (121) sowie letztere über das Ventil (111) mit Adsorber (102) und über das Ventil (112) mit Adsober (104) verbunden ist, und andererseits die Adsorber (102) und (104) über die Ventile (107) und (108) mit der Vakuumpumpe (122) sowie letztere über das Ventil (109)

...

0103070

mit Adsorber (101) und über das Ventil (110) mit Adsorber (103) verbunden ist, wobei über die Ventile (113) und (114) Produktgas gewonnen wird, während das Abgas über die Rückschlagventile (123), (124), (125) und (126) in Verbindung mit Ventil (119) entweicht.

13. Anlage nach Anspruch 12, <u>dadurch gekennzeichnet</u>, daß die Vakuumpumpe (121) die Adsorber (101) und (103) evakuiert und die evakuierten Gase (Spülgase) durch die Adsorber (102) und (104) drückt, während die Vakuumpumpe (122) die Adsorber (102) und (104) evakuiert und die evakuierten Gase (Spülgase) durch die Adsorber (101) und (103) drückt.

FIG.1

Abgas

Produktgas

Ausgangsgas

0103070

# FIG. 2

**ADS.1**

| Abgas | | Abgas | Abgas | Rückgas →ADS.2 | Produktgas Teilstrom→ADS.2 |

D.A. | Beladen | Spülen | Entspannen

Abgas    Rohgas    Rückgas   Produktgas   Produktgas

**ADS.2**

Produktgas Teilstrom→ADS.3 | Abgas | Abgas | Abgas | Rückgas →ADS.3

Entspannen | D.A. | Beladen | Spülen

Abgas   Rohgas    Rückgas   Produktgas   Produktgas

**ADS.3**

Abgas | Rückgas →ADS.4 | Produktgas Teilstrom→ADS. | Abgas | Abgas

Spülen | Entspannen | D.A. | Beladen

Produktgas   Produktgas    Abgas    Rohgas    Rückgas

**ADS.4**

Abgas | Abgas | Abgas | Rückgas →ADS.1 | Produktgas Teilstrom→ADS.1 | Abgas

Beladen | Spülen | Entspannen | D.A. | Beladen

Rohgas    Rückgas    Produktgas   Produktgas    Abgas    Rohgas

0   3   6   9   12   15   18   21   24   27   30   33   36

t [min]

D.A.: Druck-Aufbau

Rohgas = Ausgangsgas

Rückgas = Rücklaufgas

FIG. 3

Abgas

Ausgangsgas

Produktgas

0103070

# FIG.4

D.A.: Druck-Aufbau
Rohgas = Ausgangsgas
Rückgas = Rücklaufgas

FIG. 5

D.A.=Druckaufbau

FIG. 6

0103070

6 / 9

D.A. = Druckaufbau

FIG. 7

Abgas

Produktgas

7 / 9

0103070

**FIG. 8**

| | | | | |
|---|---|---|---|---|
| Adsorber 1 | Adsorptionsphase<br><br>Druck- \| Durch-<br>aufbau \| strömen | Spülphase | Desorptionsphase 1<br><br>Spülgas | Desorptionsphase 2<br><br>Produktgas |
| Adsorber 2 | Desorptionsphase 2<br><br>Produktgas | Adsorptionsphase<br><br>Druck- \| Durch-<br>aufbau \| strömen | Spülphase | Desorptionsphase 1<br><br>Spülgas |
| Adsorber 3 | Desorptionsphase 1<br><br>Spülgas | Desorptionsphase 2<br><br>Produktgas | Adsorptionsphase<br><br>Druck- \| Durch-<br>aufbau \| strömen | Spülphase |
| Adsorber 4 | Spülphase | Desorptionsphase 1<br><br>Spülgas | Desorptionsphase 2<br><br>Produktgas | Adsorptionsphase<br><br>Druck- \| Durch-<br>aufbau \| strömen |
| Steuerzeiten | ←— T1 —→ | ←— T2 —→ | ←— T3 —→ | ←— T4 —→ |
| | 1. Zyklus | | | |

FIG.9

9/9

0103070

| Schalt-ventile: | | | | |
|---|---|---|---|---|
| 18 | | | | |
| 17 | | | | |
| 16 | | | | |
| 15 | | | | |
| 14 | | | | |
| 13 | | | | |
| 12 | | | | |
| 11 | | | | |
| 10 | | | | |
| 9 | | | | |
| 8 | | | | |
| 7 | | | | |
| 6 | | | | |
| 5 | | | | |
| Steuerzeiten | T1 | T2 | T3 | T4 |